(19)

Europäisches
Patentamt
European
Patent Office
Office européen
des brevets

(11) **EP 2 803 731 A1**

(12) **EUROPEAN PATENT APPLICATION**
published in accordance with Art. 153(4) EPC

(43) Date of publication:
**19.11.2014 Bulletin 2014/47**

(21) Application number: **13736377.6**

(22) Date of filing: **11.01.2013**

(51) Int Cl.:
*C12P 7/06* (2006.01)          *C12P 7/18* (2006.01)
*C12P 7/56* (2006.01)

(86) International application number:
**PCT/JP2013/050438**

(87) International publication number:
**WO 2013/105653 (18.07.2013 Gazette 2013/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2012   JP 2012005257**

(71) Applicant: **Toray Industries, Inc.
Tokyo, 103-8666 (JP)**

(72) Inventors:
• **ISOBE, Kyohei**
  **Kamakura-shi**
  **Kanagawa 248-8555 (JP)**
• **WATANABE, Shiomi**
  **Kamakura-shi**
  **Kanagawa 248-8555 (JP)**

• **KOBAYASHI, Koji**
  **Kamakura-shi**
  **Kanagawa 248-8555 (JP)**
• **SAWAI, Kenji**
  **Kamakura-shi**
  **Kanagawa 248-8555 (JP)**
• **NA, Kyungsu**
  **Kamakura-shi**
  **Kanagawa 248-8555 (JP)**
• **HIRAMATSU, Shingo**
  **Kamakura-shi**
  **Kanagawa 248-8555 (JP)**
• **YAMADA, Katsushige**
  **Kamakura-shi**
  **Kanagawa 248-8555 (JP)**

(74) Representative: **Kador & Partner
Corneliusstraße 15
80469 München (DE)**

(54)   **METHOD FOR PRODUCING CHEMICAL SUBSTANCE**

(57)    [PROBLEMS] To provide a method for producing a chemical product with a high yield using a mixed sugar of hexose and pentose as a fermentation feedstock.

[MEANS FOR SOLUTION] A method for producing a chemical product by continuous fermentation, which method comprises filtering a culture liquid of a microorganism(s) through a separation membrane; retaining unfiltered liquid in, or refluxing unfiltered liquid to, the culture liquid; adding a fermentation feedstock to the culture liquid; and recovering a product in the filtrate, wherein the fermentation feedstock contains pentose and hexose, and wherein the microorganism(s) has/have a pathway in which pentose isomerase is used to metabolize pentose, was provided.

**EP 2 803 731 A1**

**Description**

TECHNICAL FIELD

The present invention relates to a method for producing a chemical product by continuous fermentation using a fermentation feedstock containing hexose and pentose.

BACKGROUND ART

As the problem of carbon dioxide emission into the atmosphere and the energy problem have been actualized, biomass-derived chemical products represented by biodegradable polymer materials such as lactic acid and biofuels such as ethanol have attracted stronger attention as products with sustainability and life cycle assessment (LCA) capability. These biodegradable polymer materials and biofuels are generally produced as fermentation products from microorganisms using as a fermentation feedstock glucose, which is a hexose, purified from edible biomass such as maize. However, use of edible biomass may cause a rise in its price because of competition with food, resulting in unstable supply of the feedstock. In view of this, attempts are being made to use sugars derived from non-edible biomass such as rice straw as a fermentation feedstock for microorganisms (see Patent Document 1).

In cases where a sugar derived from non-edible biomass is used as a fermentation feedstock, cellulose, hemicellulose and the like contained in the non-edible biomass are decomposed into sugars by a saccharifying enzyme. In this process, not only hexoses such as glucose, but also pentoses such as xylose are obtained, and as a consequence a mixed sugar of hexose and pentose is used as a fermentation feedstock if a sugar derived from non-edible biomass is used as a fermentation feedstock for a microorganism (see Patent Document 1).

In cases where a mixed sugar of hexose and pentose is used, a microorganism having not only a metabolic pathway for hexose but also a metabolic pathway for pentose needs to be used. Several pathways that metabolize pentose are known. In a pentose metabolic pathway in which pentose reductase acts on pentose to produce pentol in a first step of the pathway, the pentol hardly flows into the following steps of the pathway, and therefore the pentol accumulates in the culture liquid (see Non-patent Document 1). On the other hand, in a pentose metabolic pathway in which pentose isomerase acts on pentose in a first step of the pathway, pentol is not produced, and therefore the fermentation yield in this pathway is higher than in the pathway dependent on the action of pentose reductase (see Non-patent Document 1).

As a fermentation method in which a sugar derived from non-edible biomass, which is a mixed sugar of hexose and pentose, is used as a fermentation feedstock for a microorganism, continuous fermentation may be employed, but the fermentation yield actually achieved by continuous fermentation has not been studied (see Patent Document 2). On the other hand, as known in the art, in the case where continuous fermentation is performed using a microorganism having pentose isomerase and using a mixed sugar of hexose and pentose as a fermentation feedstock, the fermentation yield is lower than the case of batch fermentation (see Non-patent Document 2). Thus, according to the common technical knowledge, in order to improve the fermentation yield in continuous fermentation in which a mixed sugar of hexose and pentose is used as a fermentation feedstock for a microorganism having pentose isomerase, it has been thought that the microorganism has to be improved by genetic modification such as blocking of a biosynthetic pathway for a by-product.

PRIOR ART DOCUMENTS

PATENT DOCUMENT

Patent Document 1: WO2010/067785

NON-PATENT DOCUMENTS

Non-patent Document 1: Kaisa Karhumaa, Rosa Garcia Sanchez, Barbel Hahn-Hagerdal, Marie-F Gorwa-Grauslund, Comparison of the xylose reductase-xylitol dehydrogenase and the xylose isomerase pathways for xylose fermentation by recombinant Saccharomyces cerevisiae, Microbial Cell Factories, 6:5, (2007) Non-patent Document 2: Do Yun Kim, Seong Chun Yim, Pyung Cheon Lee, Woo Gi Lee, Sang Yup Lee, Ho Nam Chang, Batch and continuous fermentation of succinic acid from wood hydrolysate by Mannheimia succiniciproducens MBEL55E, Enzyme and Microbial Technology, 35, (2004), 648-653.

## SUMMARY OF THE INVENTION

### PROBLEMS TO BE SOLVED BY THE INVENTION

It is known that, in fermentation production of biodegradable polymer materials and biofuels, continuous fermentation in which a mixed sugar of hexose and pentose is used as a fermentation feedstock for a microorganism having pentose isomerase results in a low fermentation yield. In view of this, the present invention aims to increase the fermentation yield in continuous fermentation that is performed using a mixed sugar of hexose and pentose as a fermentation feedstock for a microorganism having a pathway in which pentose isomerase is used to metabolize pentose.

### MEANS FOR SOLVING THE PROBLEMS

As a result of intensive study to solve the above-described problem, the present inventors discovered that the problem can be solved by a method for producing a chemical product by continuous fermentation using a mixed sugar of hexose and pentose as a fermentation feedstock for a microorganism having a pathway in which pentose isomerase is used to metabolize pentose, wherein the continuous fermentation is performed using a separation membrane, thereby reaching the present invention.

That is, the present invention is as described in (1) to (6) below.

(1) A method for producing a chemical product by continuous fermentation, the method comprising filtering a culture liquid of a microorganism(s) through a separation membrane; retaining unfiltered liquid in, or refluxing unfiltered liquid to, the culture liquid; adding a fermentation feedstock to the culture liquid; and recovering a product in the filtrate, wherein the fermentation feedstock comprises pentose and hexose, and wherein the microorganism(s) has/have a pathway in which pentose isomerase is used to metabolize pentose.

(2) The method for producing a chemical product according to (1), comprising performing continuous fermentation under conditions where the oxygen transfer coefficient (Kla) is not more than 150 h$^{-1}$.

(3) The method for producing a chemical product according to (1) or (2), wherein the weight ratio between the hexose and pentose contained in the fermentation feedstock is 1:9 to 9:1.

(4) The method for producing a chemical product according to (1) or (2), wherein the fermentation feedstock comprises a biomass-derived sugar liquid.

(5) The method for producing a chemical product according to any one of (1) to (4), wherein the pentose isomerase is xylose isomerase.

(6) The method for producing a chemical product according to any one of (1) to (5), wherein the pentose is xylose.

### EFFECT OF THE INVENTION

By the present invention, a chemical product can be produced with a high yield in spite of use of a mixed sugar of hexose and pentose as a fermentation feedstock for a microorganism having a pathway in which pentose isomerase is used to metabolize pentose.

### BEST MODE FOR CARRYING OUT THE INVENTION

The present invention is a method for fermentation production of a chemical product by culturing a microorganism(s) using a fermentation feedstock, which method comprises filtering a culture liquid through a separation membrane; retaining unfiltered liquid in, or refluxing unfiltered liquid to, the culture liquid; adding a fermentation feedstock to the culture liquid; and recovering a product in the filtrate, thereby performing continuous fermentation, wherein a microorganism(s) having a pathway in which pentose isomerase is used to metabolize pentose is/are used, and the fermentation feedstock comprises hexose and pentose.

Five-carbon sugar, also called pentose, has 5 carbons constituting the sugar. Pentose can be classified into aldopentose, which has an aldehyde group at the 1-position, and ketopentose, which has a ketone group at the 2-position. Examples of aldopentose include xylose, arabinose, ribose and lyxose, and examples of ketopentose include ribulose and xylulose. The pentose used in the present invention may be any pentose as long as it can be metabolized by a microorganism, and, in view of the abundance in nature, availability and the like, xylose and arabinose are preferred, and xylose is more preferred.

Six-carbon sugar, also called hexose, has 6 carbons constituting the sugar. Hexose can be classified into aldose, which has an aldehyde group at the 1-position, and ketose, which has a ketone group at the 2-position. Examples of aldose include glucose, mannose, galactose, allose, gulose and talose, and examples of ketose include fructose, psicose and sorbose. The hexose used in the present invention may be any hexose as long as it can be metabolized by a microorganism, and, in view of the abundance in nature, availability and the like, glucose, mannose and galactose are preferred, and glucose is more preferred.

The mixed sugar used in the present invention is not limited, and the mixed sugar is preferably a sugar liquid derived from a cellulose-containing biomass that is known to contain both hexose and pentose. Examples of the cellulose-containing biomass include herbaceous biomasses such as bagasse, switchgrass, corn stover, rice straw and wheat straw; and woody biomasses such as trees and waste building materials. Cellulose-containing biomasses contain cellulose or hemicellulose, which are polysaccharides produced by dehydration condensation of sugars. By hydrolyzing such polysaccharides, sugar liquids which may be used as fermentation feedstocks are produced. The method for preparing the sugar liquid derived from a cellulose-containing biomass may be any method, and examples of disclosed methods for producing such a sugar include a method in which a sugar liquid is produced by acid hydrolysis of a biomass using concentrated sulfuric acid (JP H11-506934 A, JP 2005-229821 A), and a method in which a biomass is subjected to hydrolysis treatment with dilute sulfuric acid and then enzymatically treated with cellulase and/or the like to produce a sugar liquid (A. Aden et al., "Lignocellulosic Biomass to Ethanol Process Design and Economics Utilizing Co-Current Dilute Acid Prehydrolysis and Enzymatic Hydrolysis for Corn Stover" NREL Technical Report (2002)). Further, examples of disclosed methods in which no acids are used include a method in which a biomass is hydrolyzed using subcritical water at about 250 to 500°C to produce a sugar liquid (JP 2003-212888 A), a method in which a biomass is subjected to subcritical water treatment and then enzymatically treated to produce a sugar liquid (JP 2001-95597 A), and a method in which a biomass is subjected to hydrolysis treatment with pressurized hot water at 240 to 280°C and then enzymatically treated to produce a sugar liquid (JP 3041380 B). These treatments may be followed by purification of the obtained sugar liquid. An example of the method is disclosed in WO2010/067785.

The weight ratio between the pentose and the hexose contained in the mixed sugar is not limited, and preferably 1:9 to 9:1 as represented by the ratio of (pentose):(hexose) in terms of the weight ratio between pentose and hexose in the mixed sugar. This is the sugar ratio for cases where the mixed sugar is assumed to be a sugar liquid derived from a cellulose-containing biomass.

The total sugar concentration in the fermentation feedstock used in the present invention is not limited, and preferably as high as possible within the range in which production of the chemical product by the microorganism(s) is not inhibited. More specifically, the concentration of the carbon source in the culture medium is preferably 15 to 500 g/l, more preferably 20 to 300 g/l. In cases where the total sugar concentration is not more than 15 g/l, the effect of improving the yield from pentose may decrease. Further, in cases where the total sugar concentration is low, the production efficiency of the chemical product also decreases.

The hexose concentration in the fermentation feedstock used in the present invention is not limited as long as the total sugar concentration and the ratio between pentose and hexose are within the ranges described above. By use of the method for producing a chemical product of the present invention, a good yield can be obtained even with a mixed sugar liquid containing hexose at a concentration of not less than 5 g/L.

The fermentation feedstock used in the present invention may preferably be a usual liquid medium containing a carbon source, nitrogen source, inorganic salt, and if necessary, an organic micronutrient(s) such as an amino acid(s) and vitamin(s).

Examples of the nitrogen source used in the present invention include ammonia gas, aqueous ammonia, ammonium salts, urea and nitric acid salts, and other organic nitrogen sources used supplementarily such as oilcakes, soybean-hydrolyzed liquids, casein digests, other amino acids, vitamins, corn steep liquors, yeasts or yeast extracts, meat extracts, peptides such as peptones, and cells of various fermentation microorganisms and hydrolysates thereof. Examples of inorganic salts that may be added as appropriate include phosphoric acid salts, magnesium salts, calcium salts, iron salts and manganese salts.

In cases where the microorganism(s) used in the present invention require(s) a specific nutrient for its/their growth, the nutrient is added as a preparation or as a natural product containing the nutrient. An anti-forming agent is added as required. In the present invention, the culture liquid means a liquid obtained as a result of growth of a microorganism(s) in a fermentation feedstock. The composition of the fermentation feedstock to be added may be changed as appropriate from the composition of the fermentation feedstock used at the beginning of the culture, such that the productivity of the chemical product of interest increases.

Microorganisms that may be used for the method for producing a chemical product of the present invention are explained below.

The present invention is characterized in that a microorganism(s) having a pathway in which pentose isomerase is used to metabolize pentose is/are used. Pentose isomerase is defined as an enzyme that catalyzes isomerization of aldopentose and ketopentose, both pentoses which are constitutional isomers of each other. The pentose isomerase used in the present invention is not limited as long as it has an activity to catalyze direct isomerization of pentose, and examples of the pentose isomerase include xylose isomerase (EC 5.3.1.5) and arabinose isomerase (EC 5.3.1.3). For example, xylose isomerase is an enzyme that catalyzes direct isomerization from D-xylose (aldopentose) to D-xylulose (ketopentose) and/or the reverse isomerization thereof, and also known as D-xylose ketoisomerase. The direct isomerization means the single-step isomerization catalyzed by pentose isomerase, which is different from the two-step conversion that is catalyzed by pentose reductase and pentol dehydrogenase, which conversion proceeds via a sugar alcohol intermediate.

The microorganism(s) used in the present invention is/are not limited as long as the microorganism(s) has/have a pathway that uses pentose isomerase to metabolize pentose. A microorganism(s) that originally has/have pentose isomerase may be used, or a microorganism(s) prepared by introducing a pentose isomerase gene into an appropriate host such that the pentose isomerase functions may be used. The microorganism(s) used may a microorganism(s) isolated from the natural environment, or a microorganism(s) whose properties are partially modified by mutation or genetic recombination. Further, a microorganism(s) having an enzyme with an enhanced activity to catalyze phosphorylation of ketopentose may be used. Examples of the enzyme having an activity to catalyze phosphorylation of ketopentose include xylulokinase (EC 2.7.1.17) and ribulokinase (EC 2.7.1.16).

Examples of the microorganism having a pathway that uses pentose isomerase to metabolize pentose include enterobacteria such as *Clostridium, Enterobacter, Escherichia, Klebsiella, Bacteroides* and *Erwinia;* lactic acid bacteria such as *Lactobacillus;* actinomycetes such as *Actinoplanes, Arthrobacter* and *Streptomyces;* eumycetes such as *Piromyces* and *cyllamyces;* and microorganisms belonging to *Bacillus, Paenibacillus, Aerobacter, Ampullariella, Staphylococcus, Thermoanaerobacter* or *Thermus.* Specific examples of the microorganism include microorganisms belonging to *Escherichia, Bacillus* or *Paenibacillus* and more specific examples of the microorganism include *Escherichia coli, Bacillus coagulans* and *Paenibacillus polymyxa.* The fact that these have pentose isomerase can be easily known by searching databases published on the web such as those of KEGG (Kyoto Encyclopedia of Genes and Genomes) and NCBI (National Center for Biotechnology Information). Further, whether a microorganism whose information is not available in databases has pentose isomerase or not can be known by measuring the enzyme activity as described below.

The pentose isomerase activity can be confirmed by performing enzymatic reaction in the presence of pentose, and then measuring the pentol produced by isomerization by HPLC or the like. For example, xylose isomerase activity can be measured by the method disclosed in JP 2008-79564 A. A similar method may be used also for arabinose isomerase.

Examples of the microorganism prepared by introducing a pentose isomerase gene into an appropriate host such that the pentose isomerase functions include an example disclosed in JP 2006-525029 A. The pentose isomerase gene to be introduced is not limited, and may be either genomic DNA or cDNA. Further, the gene may be a gene derived from any organism including animals, plants, fungi (yeasts, molds and the like) and bacteria. Information on such genes can be easily known by searching databases published on the web such as those by NCBI.

The porous membrane used as a separation membrane in the present invention is explained below.

The porous membrane used in the present invention is not limited as long as it has a function to separate a culture liquid obtained by culturing a microorganism(s) in a stirred culture vessel or a stirred bioreactor from the microorganism(s) by filtration. Examples of porous membranes that may be used include porous ceramic membranes, porous glass membranes, porous organic polymer membranes, metal fiber textiles, and non-woven fabrics. Among these, porous organic polymer membranes and ceramic membranes are especially preferred.

The constitution of the porous membrane used as the separation membrane in the present invention is explained below. The porous membrane used in the present invention has a separation performance and a permeability suitable for the properties and use of the liquid to be processed.

The porous membrane is preferably a porous membrane comprising a porous resin layer in view of the blocking performance, permeability and separation performance, for example, resistance to dirt.

The porous membrane comprising a porous resin layer preferably has the porous resin layer that functions as a separation functional layer on the surface of a porous base material. The porous base material supports the porous resin layer to give strength to the separation membrane.

In cases where the porous membrane used in the present invention has a porous resin layer on the surface of a porous base material, the porous base material may be impregnated with the porous resin layer or may not be impregnated with the porous resin layer, which may be selected depending on the use of the membrane.

The average thickness of the porous base material is preferably 50 μm to 3000 μm.

The porous base material is composed of an organic material and/or inorganic material etc., and an organic fiber is preferably used. Preferred examples of the porous base material include woven fabrics and non-woven fabrics composed of organic fibers such as cellulose fibers, cellulose triacetate fibers, polyester fibers, polypropylene fibers and polyethylene fibers. More preferably, a non-woven fabric is used since its density can be relatively easily controlled; it can be simply produced; and it is inexpensive.

As the porous resin layer, an organic polymer membrane may be preferably used. Examples of the material of the organic polymer membrane include polyethylene resins, polypropylene resins, polyvinyl chloride resins, polyvinylidene fluoride resins, polysulfone resins, polyethersulfone resins, polyacrylonitrile resins, cellulose resins and cellulose triacetate resins. The organic polymer membrane may be a mixture of resins containing one or more of these resins as the major component. The major component herein means that the component is contained in an amount of not less than 50% by weight, preferably not less than 60% by weight. Preferred examples of the material of the organic polymer membrane include those which can be easily formed by solutions and are excellent in physical durability and chemical resistance, such as polyvinyl chloride resins, polyvinylidene fluoride resins, polysulfone resins, polyethersulfone resins and polyacrylonitrile resins. A polyvinylidene fluoride resin or a resin containing it as the major component is most preferably used.

As the polyvinylidene fluoride resin, a homopolymer of vinylidene fluoride is preferably used. Further, as the polyvinylidene fluoride resin, a copolymer with vinyl monomers capable of copolymerizing with vinylidene fluoride is also preferably used. Examples of the vinyl monomers capable of copolymerizing with vinylidene fluoride include tetrafluoroethylene, hexafluoropropylene and ethylene fluoride trichloride.

The porous membrane that may be used as the separation membrane in the present invention is not limited as long as the microorganism(s) used for fermentation cannot pass through the membrane, and the membrane is preferably selected within the range in which secretions from the microorganism(s) used in the fermentation or particles in the fermentation feedstock do not cause clogging and the filtration performance is stably maintained for a long period. Therefore, the average pore size of the porous separation membrane is preferably not less than 0.01 $\mu$m and less than 5 $\mu$m. The average pore size is more preferably not less than 0.01 $\mu$m and less than 1 $\mu$m since, within this range, both a high blocking performance which does not allow leakage of microorganisms and a high permeability can be achieved, and the permeability can be maintained with higher accuracy and reproducibility for a long time.

In cases where the pore size is close to the size of the microorganism(s), the pores may be blocked by the microorganism(s). Therefore, the average pore size of the porous membrane is preferably less than 1 $\mu$m. In order to prevent leakage of the microorganism(s), that is, a decrease in the elimination rate of the microorganism(s), the average pore size of the porous membrane is preferably not too large as compared to the size of the microorganism(s). In cases where a microorganism having a small cell size such as a bacterium is used, the average pore size is preferably not more than 0.4 $\mu$m, more preferably less than 0.2 $\mu$m.

In some cases, the microorganism(s) may produce substances other than the chemical product of interest, e.g. substances that are likely to aggregate such as proteins and polysaccharides. Further, in some cases, death of a part of the microorganism(s) in the fermentation culture liquid may produce cell debris. In order to prevent clogging of the porous membrane due to these substances, the average pore size is still more preferably not more than 0.1 $\mu$m.

In cases where the average pore size is too small, the permeability of the porous membrane decreases, and thus an efficient operation cannot be carried out even with a clean membrane. Therefore, the average pore size of the porous membrane in the present invention is preferably not less than 0.01 $\mu$m, more preferably not less than 0.02 $\mu$m, still more preferably not less than 0.04 $\mu$m.

The average pore size can be determined by measuring the diameters of all pores which can be observed within an area of 9.2 $\mu$m $\times$ 10.4 $\mu$m under a scanning electron microscope at a magnification of 10,000$\times$, and then averaging the measured values. Alternatively, the average pore size can be determined by taking a picture of the membrane surface under a scanning electron microscope at a magnification of 10,000$\times$, and randomly selecting not less than 10 pores, preferably not less than 20 pores, followed by measuring the diameters of these pores and calculating the number average. In cases where a pore is not circular, its size can be determined by a method in which a circle whose area is equal to the area of the pore (equivalent circle) is determined using an image processing device or the like and then the diameter of the equivalent circle is regarded as the diameter of the pore.

The standard deviation $\sigma$ of the average pore size of the porous membrane used in the present invention is preferably not more than 0.1 $\mu$m. The standard deviation $\sigma$ of the average pore size is preferably as small as possible. The standard deviation $\sigma$ of the average pore size is calculated according to the (Equation 1) below, wherein N represents the number of pores observable within the above-mentioned area of 9.2 $\mu$m $\times$ 10.4 $\mu$m, $X_k$ represents the respective measured diameters, and X(ave) represents the average of the pore diameter.

[Equation 1]

$$\sigma = \sqrt{\frac{\sum_{k=1}^{N} (X_k - X(ave))^2}{N}} \quad \cdots (1)$$

In the porous membrane used in the present invention, permeability to the fermentation culture liquid is one of the important performances. As an index of the permeability, the pure water permeability coefficient of the porous membrane before use can be employed. In the present invention, the pure water permeability coefficient of the porous membrane is preferably not less than 5.6 $\times$ 10$^{-10}$ m$^3$/m$^2$/s/pa when calculated by measuring the amount of permeation of water with a head height of 1 m using purified water at a temperature of 25°C prepared with a reverse osmosis membrane. In cases where the pure water permeability coefficient is from $5.6 \times 10^{-10}$ m$^3$/m$^2$/s/pa to $6 \times 10^{-7}$ m$^3$/m$^2$/s/pa, an amount of permeation which is practically sufficient can be obtained.

In the porous membrane used in the present invention, the surface roughness is the average of the height in the direction vertical to the surface. The membrane surface roughness is a factor that influences how easily a microorganism attached to the surface of a separation membrane is detached by the effect of washing the membrane surface with flowing liquid generated by stirring or a circulating pump. The surface roughness of the porous membrane is not limited as long as it is within the range in which the microorganism(s) and other solids attached to the membrane can be detached. The surface roughness is preferably not more than 0.1 $\mu$m. In cases where the surface roughness is not more than 0.1 $\mu$m, the microorganism(s) and other solids attached to the membrane can be easily detached.

It was found that an operation that does not require excessive power for washing the membrane surface can be carried out more easily by using, more preferably, a porous membrane having a membrane surface roughness of not more than 0.1 $\mu$m, an average pore size of not less than 0.01 $\mu$m and less than 1 $\mu$m, and a pure water permeability coefficient of not less than $2 \times 10^{-9}$ m$^3$/m$^2$/s/pa. In cases where the surface roughness of the porous membrane is not more than 0.1 $\mu$m, the shear force generated on the membrane surface during filtration of the microorganism(s) can be reduced, and hence destruction of the microorganism(s) can be suppressed, and clogging of the porous membrane can also be suppressed. Thus, long-time stable filtration can be more easily carried out. Further, in cases where the surface roughness of the porous membrane is not more than 0.1 $\mu$m, continuous fermentation can be carried out with a smaller transmembrane pressure difference. Therefore, even in cases where clogging of the porous membrane has occurred, a better washing recovery performance can be obtained as compared to cases where the operation was carried out with a larger transmembrane pressure difference. Since suppression of clogging of the porous membrane allows stable continuous fermentation, the surface roughness of the porous membrane is preferably as small as possible.

The membrane surface roughness of the porous membrane herein is measured using the following atomic force microscope (AFM) under the following conditions.

- Device
Atomic force microscope ("Nanoscope IIIa", manufactured by Digital Instruments, Inc.)
- Conditions

Probe:

SiN cantilever (manufactured by Digital Instruments, Inc.)

Scanning mode:

Contact mode (measurement in air)
Underwater tapping mode (underwater measurement)

Scanning area:

10 $\mu$m $\times$ 25 $\mu$m (measurement in air)
5 $\mu$m $\times$ 10 $\mu$m (underwater measurement)

Scanning resolution:

512 $\times$ 512

- Sample preparation
When the measurement was carried out, the membrane sample was soaked in ethanol at room temperature for 15 minutes and then soaked in RO water for 24 hours to wash it, followed by drying in the air. The RO water means water prepared by filtration through a reverse osmosis membrane (RO membrane), which is a type of filtration membrane, to remove impurities such as ions and salts. The pore size of the RO membrane is not more than about 2 nm.

The membrane surface roughness $d_{rough}$ is calculated according to the (Equation 2) below based on the height of each point in the direction of the Z-axis, as determined using the atomic force microscope (AFM).
[Equation 2]

$$d_{rough} = \sum_{n=1}^{N} \frac{|Z_n - \overline{Z}|}{N} \quad \cdots (2)$$

$d_{rough}$ : Average surface roughness ($\mu$m)
$Z_n$ : Height in the direction of the Z-axis ($\mu$m)
$\overline{Z}$ : Average height in the scanned area ($\mu$m)

The shape of the porous membrane used in the present invention is preferably a flat membrane. In cases where the shape of the porous membrane is a flat membrane, its average thickness is selected depending on its use. The average thickness in the cases where the shape of the porous membrane is a flat membrane is preferably 20 $\mu$m to 5000 $\mu$m, more preferably 50 $\mu$m to 2000 $\mu$m.

Further, the shape of the porous membrane used in the present invention is preferably a hollow fiber membrane. In cases where the porous membrane is a hollow fiber membrane, the inner diameter of the hollow fiber is preferably 200 $\mu$m to 5000 $\mu$m, and the membrane thickness is preferably 20 $\mu$m to 2000 $\mu$m. A fabric or knit produced by forming organic fibers or inorganic fibers into a cylindrical shape may be contained in the hollow fiber.

The porous membrane described above can be produced by, for example, the production method described in WO2007/097260.

In another preferred embodiment, the separation membrane in the present invention may be a membrane containing at least a ceramic. The ceramic in the present invention means a substance that contains a metal oxide and was baked by heat treatment at high temperature. Examples of the metal oxide include alumina, magnesia, titania and zirconia. The separation membrane may be formed by only a metal oxide(s), or may contain silica and/or silicon carbide, and/or mullite and/or cordierite, which are compounds of silica and a metal oxide(s).

Components forming the separation membrane other than the ceramic are not limited as long as the components can form a porous body as a separation membrane.

Even in cases where the separation membrane contains a ceramic, the shape of the separation membrane is not limited, and may be any of a monolith membrane, flat membrane, tubular membrane and the like. In view of the efficiency of packing into a container, the separation membrane preferably has a columnar shape in which a penetrating hole(s) is/are formed in the longitudinal direction. In view of increasing the packing efficiency, the separation membrane is preferably a monolith membrane.

The reason why the separation membrane preferably has a penetrating hole(s) in the longitudinal direction is as follows. In cases where a separation membrane having a columnar structure is placed in a modular container to use it as a separation membrane module, modularization of the separation membrane is possible by selecting a preferred mode from the external-pressure type and the internal-pressure type, and filtration can be carried out with the module. In the present invention, the side in which the separation membrane contacts with the fermentation culture liquid is hereinafter referred to as the primary side, and the side in which a filtrate containing a chemical product is obtained by filtration is hereinafter referred to as the secondary side.

In cases where a inner-pressure type module is used, the channel in the primary side is narrow. Therefore, the output of the circulating pump during cross-flow filtration can be saved. Further, the action to discharge the suspended matter accumulated on the surface of the separation membrane is strong, and therefore the surface of the separation membrane is likely to be kept clean, which is preferred. However, in order to obtain this effect, the inner-pressure type separation membrane needs to have an inlet and an outlet for the fermentation culture liquid. The inlet and the outlet are preferably in a state where they are arranged on a straight line to form a penetrating hole since the flow resistance is small in such a case. Further, in cases where the separation membrane has a columnar shape and the penetrating hole(s) open(s) in the longitudinal direction, the container containing the separation membrane can be made thin. A thin separation membrane module is preferred in view of production and handling.

The porosity of the separation membrane is not limited, but in cases where the porosity is too low, the filtration efficiency is low; and in cases where the porosity is too high, the strength is low. In order to achieve both high filtration efficiency and high strength of the separation membrane, as well as resistance to repeated steam sterilization, the porosity is preferably 20% to 60%.

The porosity is determined according to the following equation.

$$\text{Porosity [\%]} = 100 \times (\text{wet membrane weight [g]} - \text{dry membrane weight [g]}) /$$

$$\text{specific gravity of water [g/cm}^3\text{] / (membrane volume [cm}^3\text{])}$$

The average pore size of the separation membrane is preferably 0.01 μm to 1 μm, and a membrane having an average pore size within this range is less likely to be clogged and has excellent filtration efficiency. Further, with an average pore size within the range of 0.02 μm to 0.2 μm, substances that easily cause clogging of a separation membrane, such as by-products of fermentation by the microorganism or cultured cells, including proteins and polysaccharides, and cell debris produced by death of the microorganism/cultured cells in the culture liquid, become less likely to cause clogging, which is especially preferred.

In a separation membrane having a penetrating hole(s) and a columnar structure, the outer surface is in the secondary side. Therefore, it is preferred that a modular container be provided for collecting the filtrate and that the separation membrane be packed into the container to form a module to be used. One or more separation membranes are packed into one module.

The modular container is preferably composed of a material resistant to repeated steam sterilization. Examples of the material resistant to steam sterilization include stainless steels, and ceramics having low average porosities.

Such a ceramic membrane module can be produced by, for example, the production method described in WO2012/086763, or a commercially available module may be used. Specific examples of the commercially available module include MEMBRALOX Microfiltration Membrane (Pall Corporation) and a ceramic membrane filter Cefilt MF Membrane (NGK Insulators, Ltd.).

Next, the continuous fermentation is explained below.

The continuous fermentation in the present invention is characterized in that it is continuous fermentation in which a culture liquid of a microorganism(s) is filtered through a separation membrane; unfiltered liquid is retained in, or refluxed to, the culture liquid; a fermentation feedstock is added to the culture liquid; and a product is recovered from the filtrate.

In the culture of a microorganism(s), a pH and a temperature suitable for the microorganism(s) used may be set, and the pH and the temperature are not limited as long as the microorganism(s) can be grown. The pH and the temperature are usually within the ranges of 4 to 8 and 20 to 75°C, respectively. The pH of the culture liquid is adjusted in advance to a predetermined value usually within the range of 4 to 8 using an inorganic or organic acid, alkaline substance, urea, calcium carbonate, ammonia gas or the like.

A nutrient(s) necessary for growth of the microorganism cells may be added to the culture medium to allow continuous growth of the cells. The microorganism concentration in the culture liquid is not limited as long as it is a concentration preferred for efficient production of the chemical product. A good production efficiency can be obtained by maintaining the microorganism concentration in the culture liquid at, for example, not less than 5 g/L in terms of the dry weight.

In terms of the oxygen condition for the culture in the present invention, the oxygen transfer coefficient Kla ($h^{-1}$) (hereinafter simply referred to as Kla) is preferably not more than 150 $h^{-1}$.

Kla represents the capacity to produce dissolved oxygen by transferring oxygen from the gas phase to the liquid phase in a unit time with aeration and stirring, and is defined by the Equation (3) below. (Laboratory Manual for Bioengineering. The Society for Biotechnology, Japan ed., Baifukan Co., Ltd., p. 310 (1992)).

$$dC/dt = Kla \times (C^*-C) \ldots \text{(Equation 3)}$$

In this equation, C represents the dissolved oxygen level DO (ppm) in the culture liquid; $C^*$ represents the dissolved oxygen level DO (ppm) in the state of equilibration with the gas phase in the absence of consumption of oxygen by the microorganism(s); and Kla represents the oxygen transfer coefficient ($hr^{-1}$). Since the Equation (4) below is derived from the Equation (3) above, Kla can be determined by plotting the logarithm of $C^*-C$ against the period of aeration.

$$\ln(C^*-C) = -Kla \times t \ldots \text{(Equation 4)}$$

Kla in the present invention is a value measured by the gassing-out method (dynamic method). The gassing-out method (dynamic method) means a method in which water or the culture medium to be used is placed in an aeration-stirring culture apparatus comprising a dissolved-oxygen concentration electrode inserted therein, and oxygen in the liquid is replaced by nitrogen gas to decrease the oxygen concentration in the liquid, followed by exchanging the nitrogen gas with compressed air and measuring the process of increase of dissolved oxygen at a predetermined aeration rate, stirring rate and temperature, to calculate Kla.

Kla can be set appropriately by combining an aeration condition and a stirring condition to perform culture with aeration and stirring. In such a case, the culture may be carried out either with aeration or without aeration, and the gas to be used for aeration may be changed depending on culture conditions. For example, in order to keep a low dissolved oxygen level, the container may be tightly closed to prevent aeration; aeration may be carried out with an inert gas such as nitrogen gas; or aeration may be carried out with an inert gas containing carbon dioxide gas. On the other hand, for example, in order to keep a high dissolved oxygen level, the oxygen concentration may be kept at not less than 21% by adding oxygen into the air; or the culture liquid may be pressurized.

When Kla is within the range defined in the present invention, Kla is known to be proportional to the aeration rate as shown by the Equation (5) below, at a constant stirring rate. Accordingly, the predetermined Kla employed for the culture in the present invention can be set by measuring Kla at a constant stirring rate while the aeration rate is arbitrarily changed, and plotting the obtained Kla values against the aeration rate, followed by determining the constants a and b that satisfy Equation (5).

$$Kla = a \times V + b \ldots \text{(Equation 5)}$$

In this equation, V represents the aeration rate (wm); and a and b are constants.

The microorganism that may be used in the present invention and has a pathway in which pentose isomerase is used to metabolize pentose does not require a coenzyme such as NADH for the isomerization reaction from pentose into pentol. Therefore, the pentose metabolism is not influenced by the oxygen condition. Accordingly, chemical products can be produced without delay of the pentose metabolism even under anaerobic conditions in which oxygen is not utilized. The Kla employed in the fermentation culture of the present invention is not limited, and, specifically, the Kla is preferably not more than 150 h$^{-1}$, more preferably not more than 100 h$^{-1}$, still more preferably not more than 60 h$^{-1}$, most preferably not more than 30 h$^{-1}$. By culturing a microorganism(s) having pentose isomerase under such an oxygen condition, productivity of the chemical product of interest remarkably increases.

In the method for producing a chemical product of the present invention, the transmembrane pressure difference during filtration is not limited as long as the fermentation culture liquid can be filtered. However, in cases where filtration treatment is carried out for filtration of a culture liquid through an organic polymer membrane with a transmembrane pressure difference of more than 150 kPa, the structure of the organic polymer membrane is highly likely to be destroyed, and therefore the capacity to produce a chemical product may be deteriorated. In cases where the transmembrane pressure difference is less than 0.1 kPa, a sufficient amount of permeate of the fermentation culture liquid may not be obtained, and the productivity in production of the chemical product tends to be low. Accordingly, when an organic polymer membrane is used in the method for producing a chemical product of the present invention, the transmembrane pressure difference, which is the filtration pressure, is preferably within the range of 0.1 kPa to 150 kPa since, in such a case, the amount of permeate of the fermentation culture liquid can be large, and the decrease in the capacity to produce a chemical product due to destruction of the membrane structure does not occur. Therefore, the capacity to produce a chemical product can be kept high in such a case. In cases of an organic polymer membrane, the transmembrane pressure difference is more preferably within the range of 0.1 kPa to 50 kPa, still more preferably within the range of 0.1 kPa to 20 kPa.

Also in cases where a ceramic membrane is used, the transmembrane pressure difference during filtration is not limited as long as the fermentation culture liquid can be filtered. The transmembrane pressure difference is preferably not more than 500 kPa. In cases where the operation is carried out at not less than 500 kPa, clogging of the membrane may occur to cause a trouble in the operation of continuous fermentation.

In terms of the driving force for the filtration, a siphon using the liquid level difference (hydraulic head difference) between the fermentation culture liquid and the liquid processed through the porous membrane, or a cross-flow circulating pump, may be used to generate the transmembrane pressure difference in the separation membrane. Further, as the driving force for the filtration, a suction pump may be placed in the secondary side of the separation membrane. In cases where a cross-flow circulating pump is used, the transmembrane pressure difference can be controlled by the suction pressure. The transmembrane pressure difference can also be controlled by the pressure of the gas or liquid which is used for introducing the pressure into the fermentation liquid side. In cases where such pressure control is carried out, the difference between the pressure in the fermentation liquid side and the pressure in the side of the liquid processed through the porous membrane can be regarded as the transmembrane pressure difference, and can be used for controlling the transmembrane pressure difference.

In the present invention, continuous fermentation (filtration of culture liquid) may be started after increasing the microorganism concentration by performing batch culture or fed-batch culture at an early stage of culture. Alternatively, microorganism cells may be seeded at high concentration, and continuous culture may then be carried out from the beginning of the culture. In the method for producing a chemical product of the present invention, supply of the culture medium and filtration of the culture liquid may be carried out from an appropriate timing(s). The timings of beginning of the supply of the culture medium and filtration of the culture liquid do not necessarily need to be the same. The supply of the culture medium and filtration of the culture liquid may be carried out either continuously or intermittently.

The sugar concentration in the total filtrate during continuous fermentation is preferably not more than 5 g/L in view of productivity. The sugar concentration in the filtrate can be controlled by the supply rate of the culture medium, the filtration rate of the culture liquid, and/or the sugar concentration in the culture medium.

If necessary, during the continuous fermentation in the method for producing a chemical product of the present invention, the microorganism concentration in the culture vessel may be controlled by removing a part of the culture liquid containing the microorganism(s) from the fermenter and then diluting the culture liquid in the vessel with a culture medium. For example, when the microorganism concentration in the fermenter is too high, clogging of the separation membrane is likely to occur. The clogging of the separation membrane can be avoided by removing a part of the culture liquid containing the microorganism(s) and then diluting the culture liquid in the fermenter with the culture medium. Further, the performance for producing the chemical product may change depending on the microorganism concentration in the fermenter. The production performance may be maintained by removing the microorganism(s), using the production performance as an index.

In cases where continuous fermentation is carried out according to the method for producing a chemical product of the present invention, a higher yield can be obtained, and continuous fermentation production with much higher efficiency can be achieved, as compared to cases where conventional culture is performed. The yield in the continuous culture herein is calculated according to the Equation (7) below.

$$\text{Yield (g/g)} = \text{Amount of product (g)} / \{\text{Fed sugar (g)} - \text{Unused sugar (g)}\} \ldots$$

(Equation 7)

The continuous culture apparatus used in the present invention is not limited as long as it is an apparatus for producing a chemical product by continuous fermentation in which a fermentation culture liquid of a microorganism(s) is filtered through a separation membrane and the product is recovered from the filtrate, while the unfiltered liquid is retained in, or refluxed to, the fermentation culture liquid; a fermentation feedstock is added to the fermentation culture liquid; and the product in the filtrate is recovered. Specific examples of the apparatus in which an organic polymer membrane is used include the apparatus described in WO2007/097260. Specific examples of the apparatus in which a ceramic membrane is used include the apparatus described in WO2012/086763.

The chemical product produced by the present invention is not restricted as long as it is a substance produced in a fermentation culture liquid by the above-described microorganisms. Examples of the chemical product include alcohols, organic acids, amino acids and nucleic acids, which are substances mass-produced in the fermentation industry. Examples the substances include alcohols such as ethanol, 1,3-propanediol, 1,3-butanediol, 2,3-butanediol, 1,4-butanediol, glycerol, butanol, isobutanol, 2-butanol and isopropanol; organic acids such as acetic acid, lactic acid, adipic acid, pyruvic acid, succinic acid, malic acid, itaconic acid and citric acid; nucleic acids such as nucleosides including inosine and guanosine, and nucleotides including inosinic acid and guanylic acid; and diamine compounds such as cadaverine. Among these, organic acids and alcohols are preferred, and lactic acid, ethanol and 2,3-butanediol are more preferred. Further, the present invention may also be applied to production of substances such as enzymes, antibiotics and recombinant proteins. These chemical products can be recovered from the filtrate by well-known methods (membrane separation, concentration, distillation, crystallization, extraction and the like).

EXAMPLES

The present invention will now be described concretely by way of Examples. However, the present invention is not limited to these.

(Reference Example 1) Method for Analyzing Glucose, Xylose, Xylulose, Ethanol and 2,3-Butanediol

The concentrations of glucose, xylose, xylulose, ethanol and 2,3-butanediol in the fermentation liquid were quantified under the following HPLC conditions by comparison with standard samples.

Column: Shodex SH1011 (manufactured by Showa Denko K. K.)

Mobile phase: 5 mM sulfuric acid (flow rate: 0.6 mL/min)
Reaction liquid: none
Detection method: RI (differential refractive index)
Temperature: 65°C

(Reference Example 2) Method for Analyzing Lactic Acid

Lactic acid in the fermentation liquid was quantified under the following HPLC conditions by comparison with standard samples.

Column: Shim-Pack SPR-H (manufactured by Shimadzu Corporation)
Mobile phase: 5 mM p-toluenesulfonic acid (flow rate: 0.8 mL/min)
Reaction liquid: 5 mM p-toluenesulfonic acid, 20 mM Bis-Tris, 0.1 mM EDTA-2Na (flow rate: 0.8 mL/min)
Detection method: electric conductivity
Temperature : 45°C

(Reference Example 3) Measurement of Kla

A dissolved-oxygen electrode (manufactured by Mettler-Toledo) was inserted into the culture vessel to measure the dissolved-oxygen level under each aeration/stirring condition, and Kla was determined by the dynamic method using nitrogen gas. In a culture vessel, 1.5 L of water was placed, and nitrogen gas was sufficiently blown into the water while the water temperature was controlled at 30°C or 50°C and while water was stirred at a constant rate. When the electrode value became minimum, zero calibration of the dissolved-oxygen electrode was carried out. Thereafter, the aeration gas was changed from nitrogen gas to air or nitrogen gas at a predetermined aeration rate, and changes in the dissolved-oxygen level with time were measured to determine Kla. Table 1 shows Kla obtained for various aeration/stirring conditions.

[Table 1]

| Aeration gas | Aeration rate (vvm) | Stirring rate (rpm) | 30°C $Kla$ $(h^{-1})$ | 50°C $Kla$ $(h^{-1})$ |
|---|---|---|---|---|
| Nitrogen | 0.1 | 200 | 0 | 0 |
| Air | 0.01 | 800 | 4 | 3 |
| | 0.05 | 800 | 14 | 13 |
| | 0.1 | 800 | 27 | 24 |
| | 0.2 | 800 | 57 | 52 |
| | 0.3 | 800 | 89 | 81 |
| | 0.5 | 800 | 144 | 132 |
| | 0.6 | 800 | 168 | 151 |

According to Table 1, the constants (a, b) in Equation (5) obtained by plotting the relationship between the aeration rate and Kla observed when aeration was carried out with air at a stirring rate of 800 rpm were (284, 0.49) and (257, -0.47) at 30°C and 50°C, respectively.

(Reference Example 4) Measurement of Xylose Isomerase Activity in *Escherichia coli*

The *Escherichia coli* KO11 strain was inoculated to 5 mL of a preculture medium (20 g/L glucose, 10 g/L yeast extract, 5 g/L tryptone, 5 g/L NaCl) using a platinum loop, and culture was carried out at 30°C overnight (preculture). On the next day, the preculture liquid was inoculated to 50 mL of the ethanol fermentation medium (mixed sugar) shown in Table 2 placed in a 500-mL Erlenmeyer flask, and culture was performed at 30°C for 24 hours. After recovering the culture liquid, washing was carried out twice with 200 mM maleate buffer (pH 5.5). The microorganism cells were resuspended in 200 mM maleate buffer (pH 5.5) and homogenized with a bead homogenizer (0.6 g of $\varphi$0.5-beads, 4000 rpm, 1 minute $\times$ 5 times), followed by centrifugation and recovery of the resulting supernatant, which supernatant was used as a microorganism cell extract. Thereafter, 100 $\mu$L of the microorganism cell extract was added to 900 $\mu$L of 200

mM maleate buffer (pH 5.5) supplemented with 20 mM $MgSO_4$, 1 mM $CoCl_2$, 1 mM $MnCl_2$ and 10 mM xylose, and the reaction was allowed to proceed at 35°C for 2 hours, followed by analyzing the reaction liquid by HPLC. As a result, the peak for xylose was found to be lower, and the peak for xylulose was found to be higher, as compared to those before the reaction. Thus, the xylose isomerase activity could be confirmed.

[Table 2]

Ethanol fermentation medium (mixed sugar)

| | |
|---|---|
| Glucose | 30 g |
| Xylose | 40 g |
| Yeast extract | 10 g |
| Tryptone | 5 g |
| NaCl | 5 g |

Unit (1/Liter)

(Comparative Example 1) Production of Ethanol by Batch Fermentation by *Escherichia coli* Using Hexose as Fermentation Feedstock

The *Escherichia coli* KO11 strain was cultured in 2 mL of a preculture medium (20 g/L glucose, 10 g/L yeast extract, 5 g/L tryptone, 5 g/L NaCl) in a test tube at 30°C overnight (pre-preculture). The obtained culture liquid was inoculated to 50 mL of a preculture medium placed in a 500-mL baffled Erlenmeyer flask, and culture was performed overnight (preculture). The preculture liquid was inoculated to 1 L of the ethanol fermentation medium (glucose) having the composition shown in Table 3, and batch fermentation was carried out for 16 hours under the following operating conditions while the temperature and the pH were controlled, to produce ethanol (Table 4).

Culture vessel capacity: 2 (L)
Temperature adjustment: 30 (°C)
Kla: 30 ($h^{-1}$)
pH Adjustment: adjusted to pH 6 with 5 N $Ca(OH)_2$
Sterilization: the fermentation vessel and media to be used were all subjected to high-pressure steam sterilization by autoclaving at 121 °C for 20 min.

[Table 3]

Ethanol fermentation medium (glucose)

| | |
|---|---|
| Glucose | 40 g |
| Yeast extract | 10 g |
| Tryptone | 5 g |
| NaCl | 5 g |

Unit (1/Liter)

(Comparative Example 2) Production of Ethanol by Batch Fermentation by *Escherichia coli* Using Mixed Sugar as Fermentation Feedstock

Using the ethanol fermentation medium (mixed sugar) having the composition shown in Table 2 as the culture medium, batch fermentation was carried out for 33 hours under the same conditions as in Comparative Example 1, to produce ethanol (Table 4).

(Comparative Example 3) Production of Ethanol by Continuous Fermentation by *Escherichia coli* Using Mixed Sugar as Fermentation Feedstock

Continuous fermentation was carried out using a mixed sugar as the fermentation feedstock, without use of a separation membrane. The *Escherichia coli* KO11 strain was cultured in 2 mL of a preculture medium (20 g/L glucose, 10 g/L yeast extract, 5 g/L tryptone, 5 g/L NaCl) in a test tube at 30°C overnight (pre-pre-preculture). The obtained culture liquid was inoculated to 50 mL of a preculture medium in a 500-mL baffled Erlenmeyer flask, and culture was performed overnight (pre-preculture). The pre-preculture liquid was inoculated to the ethanol fermentation medium (mixed sugar) having the composition shown in Table 2 placed in a continuous culture apparatus (the same apparatus as shown in Fig. 2 of WO2007/097260 except that the separation membrane element was eliminated), and batch fermentation was carried out for 33 hours under the operating conditions shown below while the temperature and the pH were controlled (preculture). Immediately after completion of the preculture, continuous culture was started to produce ethanol. For supplying the ethanol fermentation medium (mixed sugar) having the composition shown in Table 2 and collecting the culture liquid containing the microorganism, a Perista BioMini Pump Type AC-2120 (ATTO) was used to supply the culture medium directly to the culture vessel and to collect the culture liquid containing the microorganism directly from the culture vessel. While the rate of supplying the culture medium was controlled such that the amount of culture liquid in the culture vessel was 1.5 L at a constant rate of collection of the culture liquid containing the microorganism, ethanol production was performed for 300 hours (Table 4).

Culture vessel capacity: 2 (L)
Temperature adjustment: 30 (°C)
Kla: 30 (h$^{-1}$)
pH Adjustment: adjusted to pH 6 with 5 N Ca(OH)$_2$
Rate of collection of the fermentation liquid: 2 L/day
Sterilization: the fermentation vessel and media to be used were all subjected to high-pressure steam sterilization by autoclaving at 121°C for 20 min.

(Example 1) Production of Ethanol by Continuous Fermentation by *Escherichia coli* Using Mixed Sugar as Fermentation Feedstock, with Use of Separation Membrane 1

Using a mixed sugar as the fermentation feedstock, continuous fermentation was carried out with use of a separation membrane. The *Escherichia coli* KO11 strain was cultured in 2 mL of a preculture medium (20 g/L glucose, 10 g/L yeast extract, 5 g/L tryptone, 5 g/L NaCl) in a test tube at 30°C overnight (pre-pre-preculture). The obtained culture liquid was inoculated to 50 mL of a preculture medium placed in a 500-mL baffled Erlenmeyer flask, and culture was performed overnight (pre-preculture). The pre-preculture liquid was inoculated to the ethanol fermentation medium (mixed sugar) having the composition shown in Table 2 placed in a continuous fermentation apparatus equipped with an integrated membrane having the properties shown below (the apparatus shown in Fig. 2 of WO2007/097260), and batch fermentation was carried out for 24 hours under the operating conditions shown below while the temperature and the pH were controlled (preculture). Immediately after completion of the preculture, continuous culture was started to produce ethanol. For supplying the ethanol fermentation medium (mixed sugar) having the composition shown in Table 2 and filtering the culture liquid, a Perista BioMini Pump Type AC-2120 (ATTO) was used. The culture medium was directly supplied to the culture vessel, and the culture liquid was filtered through an element having an immobilized separation membrane. While the rate of supplying the culture medium was controlled such that the amount of culture liquid in the culture vessel was 1.5 L at a constant rate of filtration of the culture liquid, and while the transmembrane pressure difference during filtration was allowed to change within the range of 0.1 to 19.8 kPa, continuous fermentation was performed for 290 hours to produce ethanol (Table 4).

Fermentation reaction vessel capacity: 2 (L)
Separation membrane used: polyvinylidene fluoride filtration membrane
Effective filtration area of the membrane separation element: 120 cm$^2$
Pure water permeation coefficient of the separation membrane: 50 × 10$^{-9}$ m$^3$/m$^2$/s/Pa
Average pore size of the separation membrane: 0.1 μm
Standard deviation of the average pore size: ±0.035 μm
Surface roughness of the separation membrane: 0.06 μm
Temperature adjustment: 30 (°C)
Kla: 30 (h$^{-1}$)
pH Adjustment: adjusted to pH 6 with 5 N Ca(OH)$_2$
Rate of collection of the fermentation liquid: 2 L/day
Sterilization: the fermentation vessel comprising the separation membrane element and media to be used were all subjected to high-pressure steam sterilization by autoclaving at 121 °C for 20 min.

(Example 2) Production of Ethanol by Continuous Fermentation by *Escherichia coli* Using Mixed Sugar as Fermentation Feedstock, with Use of Separation Membrane 2

Using the *Escherichia coli* KO11 strain, continuous fermentation was carried out with use of a separation membrane. The continuous fermentation was performed for 300 hours under the same conditions as in Example 1 except that Kla was set to 0, to produce ethanol (Table 4).

(Example 3) Production of Ethanol by Continuous Fermentation by *Escherichia coli* Using Mixed Sugar as Fermentation Feedstock, with Use of Separation Membrane 3

Using the *Escherichia coli* KO11 strain, continuous fermentation was carried out with use of a separation membrane. The continuous fermentation was performed for 280 hours under the same conditions as in Example 1 except that Kla was set to 60, to produce ethanol (Table 4).

(Example 4) Production of Ethanol by Continuous Fermentation by *Escherichia coli* Using Mixed Sugar as Fermentation Feedstock, with Use of Separation Membrane 4

Using the *Escherichia coli* KO11 strain, continuous fermentation was carried out with use of a separation membrane. The continuous fermentation was performed for 290 hours under the same conditions as in Example 1 except that Kla was set to 100, to produce ethanol (Table 4).

(Example 5) Production of Ethanol by Continuous Fermentation by *Escherichia coli* Using Mixed Sugar as Fermentation Feedstock, with Use of Separation Membrane 5

Using the *Escherichia coli* KO11 strain, continuous fermentation was carried out with use of a separation membrane. The continuous fermentation was performed for 270 hours under the same conditions as in Example 1 except that Kla was set to 150, to produce ethanol (Table 4).

[Table 4]

| | Comparative Example 1 | Comparative Example 2 | Comparative Example 3 | Example 1 | Example 2 | Example 3 | Example 4 | Example 5 |
|---|---|---|---|---|---|---|---|---|
| Fermentation period (hr) | 16 | 33 | 300 | 290 | 300 | 280 | 290 | 270 |
| Total glucose fed (g) | 40 | 30 | 750 | 725 | 750 | 700 | 725 | 675 |
| Total xylose fed (g) | 0 | 40 | 1000 | 967 | 1000 | 933 | 967 | 900 |
| Total production of ethanol (g) | 17.5 | 15.4 | 250 | 643 | 781 | 572 | 474 | 378 |
| Unused glucose (g) | 0 | 0 | 0 | 0 | 0 | 0 | 0 | 0 |
| Unused xylose (g) | 0 | 0 | 435 | 0 | 14 | 0 | 0 | 0 |
| Yield (g/g) | 0.35 | 0.22 | 0.19 | 0.38 | 0.45 | 0.35 | 0.28 | 0.24 |

(Reference Example 5) Measurement of Xylose Isomerase Activity in *Paenibacillus polymyxa*

The *Paenibacillus polymyxa* ATCC12321 strain was inoculated to 5 mL of a preculture medium (5 g/L glucose, 5 g/L peptone, 3 g/L yeast extract, 3 g/L malt extract) using a platinum loop, and culture was carried out at 30°C overnight (preculture). On the next day, the preculture liquid was inoculated to 50 mL of the 2,3-butanediol fermentation medium 1 (mixed sugar) shown in Table 5 placed in a 500-mL Erlenmeyer flask, and culture was performed at 30°C for 24 hours. The recovered culture liquid was washed twice with 200 mM maleate buffer (pH 5.5). The microorganism cells were resuspended in 200 mM maleate buffer (pH 5.5) and homogenized with a bead homogenizer (0.6 g of $\varphi$0.5-beads, 4000 rpm, 1 minute $\times$ 5 times), followed by centrifugation and recovery of the resulting supernatant, which supernatant was used as a microorganism cell extract. Thereafter, 100 $\mu$L of the microorganism cell extract was added to 900 $\mu$L of 200 mM maleate buffer (pH 5.5) supplemented with 20 mM $MgSO_4$, 1 mM $CoCl_2$, 1 mM $MnCl_2$ and 10 mM xylose, and the reaction was allowed to proceed at 35°C for 2 hours, followed by analyzing the reaction liquid by HPLC. As a result, the peak for xylose was found to be lower, and the peak for xylulose was found to be higher, as compared to those before the reaction. Thus, the xylose isomerase activity could be confirmed.

[Table 5]

2,3-Butanediol fermentation medium 1 (mixed sugar)

| | |
|---|---|
| Glucose | 20 g |
| Xylose | 40 g |
| Yeast extract | 13.1 g |
| Ammonium sulfate | 5.8 g |
| $KH_2PO_4$ | 1.75 g |
| $K_2HPO_4$ | 9.2 g |
| $(NH_4)_2HPO_4$ | 2.9 g |
| $CaCl_2 \cdot 2H_2O$ | 8.8 mg |
| $FeSO_4 \cdot 7H_2O$ | 44 mg |
| $MnSO_4 \cdot 5H_2O$ | 1.28 mg |
| $ZnSO_4 \cdot 7H_2O$ | 0.9 mg |
| $MgSO_4 \cdot 7H_2O$ | 219 mg |
| $EDTA \cdot 2Na$ | 44 mg |

Unit (1/Liter)

(Comparative Example 4) Production of 2,3-Butanediol by Batch Fermentation by *Paenibacillus polymyxa* Using Hexose as Fermentation Feedstock

The *Paenibacillus polymyxa* ATCC12321 strain was cultured in 2 mL of a preculture medium (5 g/L glucose, 5 g/L peptone, 3 g/L yeast extract, 3 g/L malt extract) in a test tube at 30°C overnight (pre-preculture). The obtained culture liquid was inoculated to 50 mL of a preculture medium placed in a 500-mL baffled Erlenmeyer flask, and culture was performed overnight (preculture). The preculture liquid was inoculated to 1 L of the 2,3-butanediol fermentation medium (glucose) having the composition shown in Table 6, and batch fermentation was carried out for 27 hours under the following operating conditions while the temperature and the pH were controlled, to produce 2,3-butanediol (Table 8).

Culture vessel capacity: 2 (L)
Temperature adjustment: 30 (°C)
Kla: 30 ($h^{-1}$)
pH Adjustment: adjusted to pH 6.5 with 5 N $Ca(OH)_2$
Sterilization: the fermentation vessel and media to be used were all subjected to high-pressure steam sterilization by autoclaving at 121°C for 20 min.

[Table 6]

2,3-Butanediol fermentation medium (glucose)

| | |
|---|---|
| Glucose | 50 g |
| Yeast extract | 13.1 g |

(continued)

| 2,3-Butanediol fermentation medium (glucose) | |
|---|---|
| Ammonium sulfate | 5.8 g |
| $KH_2PO_4$ | 1.75 g |
| $K_2HPO_4$ | 9.2 g |
| $(NH_4)_2HPO_4$ | 2.9 g |
| $CaCl_2 \cdot 2H_2O$ | 8.8 mg |
| $FeSO_4 \cdot 7H_2O$ | 44 mg |
| $MnSO_4 \cdot 5H_2O$ | 1.28 mg |
| $ZnSO_4 \cdot 7H_2O$ | 0.9 mg |
| $MgSO_4 \cdot 7H_2O$ | 219 mg |
| EDTA·2Na | 44 mg |

Unit (1/Liter)

(Comparative Example 5) Production of 2,3-Butanediol by Batch Fermentation by *Paenibacillus polymyxa* Using Mixed Sugar as Fermentation Feedstock

Using the 2,3-butanediol fermentation medium 1 (mixed sugar) having the composition shown in Table 5 as the culture medium, batch fermentation was carried out for 50 hours under the same conditions as in Comparative Example 4, to produce 2,3-butanediol (Table 8).

(Comparative Example 6) Production of 2,3-butanediol by Continuous Fermentation by *Paenibacillus polymyxa* Using Mixed Sugar as Fermentation Feedstock

Continuous fermentation was carried out using a mixed sugar as the fermentation feedstock, without use of a separation membrane. The *Paenibacillus polymyxa* ATCC 12321 strain was cultured in 2 mL of a preculture medium (5 g/L glucose, 5 g/L peptone, 3 g/L yeast extract, 3 g/L malt extract) in a test tube at 30°C overnight (pre-pre-preculture). The obtained culture liquid was inoculated to 50 mL of a preculture medium placed in a 500-mL baffled Erlenmeyer flask, and culture was performed overnight (pre-preculture). The pre-preculture liquid was inoculated to the 2,3-butanediol fermentation medium 1 (mixed sugar) having the composition shown in Table 5 placed in a continuous culture apparatus (the same apparatus as shown in Fig. 2 of WO2007/097260 except that the separation membrane element was eliminated), and batch fermentation was carried out for 30 hours under the operating conditions shown below while the temperature and the pH were controlled (preculture). Immediately after completion of the preculture, continuous culture was started to produce 2,3-butanediol. For supplying the 2,3-butanediol fermentation medium 2 (mixed sugar) having the composition shown in Table 7 and collecting the culture liquid containing the microorganism, a Perista BioMini Pump Type AC-2120 (ATTO) was used to supply the culture medium directly to the culture vessel and to collect the culture liquid containing the microorganism directly from the culture vessel. While the rate of supplying the culture medium was controlled such that the amount of culture liquid in the culture vessel was 1.5 L at a constant rate of collection of the culture liquid containing the microorganism, production of 2,3-butanediol was performed for 280 hours (Table 8).

Culture vessel capacity: 2 (L)
Temperature adjustment: 30 (°C)
Kla: 30 ($h^{-1}$)
pH Adjustment: adjusted to pH 6.5 with 5 N $Ca(OH)_2$
Rate of collection of the fermentation liquid: 1 L/day
Sterilization: the fermentation vessel and media to be used were all subjected to high-pressure steam sterilization by autoclaving at 121 °C for 20 min.

[Table 7]

| 2,3-Butanediol fermentation medium 2 (mixed sugar) | |
|---|---|
| Glucose | 40 g |
| Xylose | 80 g |
| Yeast extract | 13.1 g |
| Ammonium sulfate | 5.8 g |

(continued)

| 2,3-Butanediol fermentation medium 2 (mixed sugar) | |
|---|---|
| $KH_2PO_4$ | 1.75 g |
| $K_2HPO_4$ | 9.2 g |
| $(NH_4)_2HPO_4$ | 2.9 g |
| $CaCl_2 \cdot 2H_2O$ | 8.8 mg |
| $FeSO_4 \cdot 7H_2O$ | 44 mg |
| $MnSO_4 \cdot 5H_2O$ | 1.28 mg |
| $ZnSO_4 \cdot 7H_2O$ | 0.9 mg |
| $MgSO_4 \cdot 7H_2O$ | 219 mg |
| $EDTA \cdot 2Na$ | 44 mg |

Unit (1/Liter)

(Example 6) Production of 2,3-Butanediol by Continuous Fermentation by *Paenibacillus polymyxa* Using Mixed Sugar as Fermentation Feedstock, with Use of Separation Membrane 1

Using a mixed sugar as the fermentation feedstock, continuous fermentation was carried out with use of a separation membrane. The *Paenibacillus polymyxa* ATCC12321 strain was cultured in 2 mL of a preculture medium (5 g/L glucose, 5 g/L peptone, 3 g/L yeast extract, 3 g/L malt extract) in a test tube at 30°C overnight (pre-pre-preculture). The obtained culture liquid was inoculated to 50 mL of a preculture medium placed in a 500-mL baffled Erlenmeyer flask, and culture was performed overnight (pre-preculture). The pre-preculture liquid was inoculated to the 2,3-butanediol fermentation medium 1 (mixed sugar) having the composition shown in Table 5 placed in a continuous culture apparatus (the apparatus shown in Fig. 2 of WO2007/097260), and batch fermentation was carried out for 30 hours under the operating conditions shown below while the temperature and the pH were controlled (preculture). Immediately after completion of the preculture, continuous culture was started to produce 2,3-butanediol. For supplying the 2,3-butanediol fermentation medium 2 (mixed sugar) having the composition shown in Table 7 and filtering the culture liquid, a Perista BioMini Pump Type AC-2120 (ATTO) was used. The culture medium was directly supplied to the culture vessel, and the culture liquid was filtered through an element having an immobilized separation membrane. While the rate of supplying the culture medium was controlled such that the amount of culture liquid in the culture vessel was 1.5 L at a constant rate of filtration of the culture liquid, and while the transmembrane pressure difference during filtration was allowed to change within the range of 0.1 to 19.4 kPa, continuous fermentation was performed for 280 hours to produce 2,3-butanediol (Table 8).

Fermentation reaction vessel capacity: 2 (L)
Separation membrane used: polyvinylidene fluoride filtration membrane
Effective filtration area of the membrane separation element: 120 cm$^2$
Pure water permeation coefficient of the separation membrane: 50 $\times$ 10$^{-9}$ m$^3$/m$^2$/s/Pa
Average pore size of the separation membrane: 0.1$\pm$0.035 $\mu$m
Surface roughness of the separation membrane: 0.06 $\mu$m
Temperature adjustment: 30 (°C)
Kla: 30 (h$^{-1}$)
pH Adjustment: adjusted to pH 6.5 with 5 N Ca(OH)$_2$
Rate of collection of the fermentation liquid: 1 L/day
Sterilization: the fermentation vessel comprising the separation membrane element and media to be used were all subjected to high-pressure steam sterilization by autoclaving at 121 °C for 20 min.

(Example 7) Production of 2,3-Butanediol by Continuous Fermentation by *Paenibacillus polymyxa* Using Mixed Sugar as Fermentation Feedstock, with Use of Separation Membrane 2

Using the *Paenibacillus polymyxa* ATCC12321 strain, continuous fermentation was carried out with use of a separation membrane. The continuous fermentation was performed for 300 hours under the same conditions as in Example 1 except that Kla was set to 0, to produce 2,3-butanediol (Table 8).

(Example 8) Production of 2,3-Butanediol by Continuous Fermentation by *Paenibacillus polymyxa* Using Mixed Sugar as Fermentation Feedstock, with Use of Separation Membrane 3

Using the *Paenibacillus polymyxa* ATCC12321 strain, continuous fermentation was carried out with use of a separation membrane. The continuous fermentation was performed for 270 hours under the same conditions as in Example 1 except that Kla was set to 60, to produce 2,3-butanediol (Table 8).

(Example 9) Production of 2,3-Butanediol by Continuous Fermentation by *Paenibacillus polymyxa* Using Mixed Sugar as Fermentation Feedstock, with Use of Separation Membrane 4

Using the *Paenibacillus polymyxa* ATCC12321 strain, continuous fermentation was carried out with use of a separation membrane. The continuous fermentation was performed for 280 hours under the same conditions as in Example 1 except that Kla was set to 100, to produce 2,3-butanediol (Table 8).

(Example 10) Production of 2,3-Butanediol by Continuous Fermentation by *Paenibacillus polymyxa* Using Mixed Sugar as Fermentation Feedstock, with Use of Separation Membrane 5

Using the *Paenibacillus polymyxa* ATCC12321 strain, continuous fermentation was carried out with use of a separation membrane. The continuous fermentation was performed for 270 hours under the same conditions as in Example 1 except that Kla was set to 150, to produce 2,3-butanediol (Table 8).

[Table 8]

| | Comparative Example 4 | Comparative Example 5 | Comparative Example 6 | Example 6 | Example 7 | Example 8 | Example 9 | Example 10 |
|---|---|---|---|---|---|---|---|---|
| Fermentation period (hr) | 27 | 50 | 280 | 280 | 300 | 270 | 280 | 270 |
| Total glucose fed (g) | 50 | 20 | 467 | 467 | 500 | 450 | 467 | 450 |
| Total xylose fed (g) | 0 | 40 | 933 | 933 | 1000 | 900 | 933 | 900 |
| Total production of 2,3-butanediol (g) | 12.5 | 2.1 | 168 | 433 | 331 | 351 | 266 | 203 |
| Unused glucose (g) | 0 | 0 | 0 | 0 | 0 | 0 | 0 | 0 |
| Unused xylose (g) | 0 | 0 | 109 | 3.6 | 62 | 1.6 | 0 | 0 |
| Yield (g/g) | 0.25 | 0.035 | 0.13 | 0.31 | 0.23 | 0.26 | 0.19 | 0.15 |

(Reference Example 6) Measurement of Xylose Isomerase Activity in *Bacillus coagulans*

The *Bacillus coagulans* NBRC12714 strain was inoculated to 5 mL of a preculture medium (10 g/L polypeptone, 2 g/L yeast extract, 1 g/L magnesium sulfate 7H$_2$O, 30 g/L calcium carbonate) using a platinum loop, and culture was carried out at 50°C overnight (preculture). On the next day, the preculture liquid was inoculated to 50 mL of the L-lactic acid fermentation medium (mixed sugar) shown in Table 9 supplemented with 30 g/L calcium carbonate placed in a 500-mL Erlenmeyer flask, and culture was performed at 50°C for 24 hours. The recovered culture liquid was washed twice with 200 mM maleate buffer (pH 5.5). The microorganism cells were resuspended in 200 mM maleate buffer (pH 5.5) and homogenized with a bead homogenizer (0.6 g of φ0.5-beads, 4000 rpm, 1 minute × 5 times), followed by centrifugation and recovery of the resulting supernatant, which supernatant was used as a microorganism cell extract. Thereafter, 100 μL of the microorganism cell extract was added to 900 μL of 200 mM maleate buffer (pH 5.5) supplemented with 20 mM MgSO$_4$, 1 mM CoCl$_2$, 1 mM MnCl$_2$ and 10 mM xylose, and the reaction was allowed to proceed at 35°C for 2 hours, followed by analyzing the reaction liquid by HPLC. As a result, the peak for xylose was found to be lower, and the peak for xylulose was found to be higher, as compared to those before the reaction. Thus, the xylose isomerase activity could be confirmed.

[Table 9]
L-Lactic acid fermentation medium (mixed sugar) 1

| | |
|---|---|
| Glucose | 50 g |
| Xylose | 50 g |
| Yeast extract | 5 g |
| Ammonium sulfate | 1 g |
| K$_2$HPO$_4$ | 0.4 g |

Unit (1/Liter)

(Comparative Example 7) Production of L-Lactic Acid by Batch Fermentation by *Bacillus coagulans* Using Hexose as Fermentation Feedstock

The *Bacillus coagulans* NBRC12714 strain was cultured in 2 mL of a preculture medium (10 g/L polypeptone, 2 g/L yeast extract, 1 g/L magnesium sulfate 7H$_2$O, 30 g/L calcium carbonate) in a test tube at 50°C overnight (pre-preculture). The obtained culture liquid was inoculated to 50 mL of a preculture medium placed in a 500-mL baffled Erlenmeyer flask, and culture was performed overnight (preculture). The preculture liquid was inoculated to 1 L of the L-lactic acid fermentation medium (glucose) having the composition shown in Table 10, and batch fermentation was carried out for 96 hours under the following operating conditions while the temperature and the pH were controlled, to produce L-lactic acid (Table 13).

Culture vessel capacity: 2 (L)
Temperature adjustment: 50 (°C)
Kla: 30 (h$^{-1}$)
pH Adjustment: adjusted to pH 7 with 5 N Ca(OH)$_2$
Sterilization: the fermentation vessel and media to be used were all subjected to high-pressure steam sterilization by autoclaving at 121 °C for 20 min.

[Table 10]
L-Lactic acid fermentation medium (glucose)

| | |
|---|---|
| Glucose | 100 g |
| Yeast extract | 5 g |
| Ammonium sulfate | 1 g |
| K$_2$HPO$_4$ | 0.4 g |

Unit (1/Liter)

(Comparative Example 8) Production of L-Lactic Acid by Batch Fermentation by *Bacillus coagulans* Using Mixed Sugar as Fermentation Feedstock

Using the L-lactic acid fermentation medium (mixed sugar) having the composition shown in Table 9 as the culture medium, batch fermentation was carried out for 128 hours under the same conditions as in Comparative Example 7, to produce L-lactic acid (Table 13).

(Comparative Example 9) Production of L-Lactic Acid by Continuous Fermentation by *Bacillus coagulans* Using Mixed Sugar as Fermentation Feedstock

Continuous fermentation was carried out using a mixed sugar as the fermentation feedstock, without use of a separation membrane. The *Bacillus coagulans* NBRC12714 strain was cultured in 2 mL of a preculture medium (10 g/L polypeptone, 2 g/L yeast extract, 1 g/L magnesium sulfate 7H$_2$O, 30 g/L calcium carbonate) in a test tube at 50°C overnight (pre-pre-preculture). The obtained culture liquid was inoculated to 50 mL of a preculture medium in a 500-mL baffled Erlenmeyer flask, and culture was performed overnight (pre-preculture). The pre-preculture liquid was inoculated to the L-lactic acid fermentation medium (mixed sugar) having the composition shown in Table 9 placed in a continuous culture apparatus (the same apparatus as shown in Fig. 2 of WO2007/097260 except that the separation membrane element was eliminated), and batch fermentation was carried out for 124 hours under the operating conditions shown below while the temperature and the pH were controlled (preculture). Immediately after completion of the preculture, continuous culture was started to produce L-lactic acid. For supplying the L-lactic acid fermentation medium (mixed sugar) having the composition shown in Table 9 and collecting the culture liquid containing the microorganism, a Perista BioMini Pump Type AC-2120 (ATTO) was used to supply the culture medium directly to the culture vessel and to collect the culture liquid containing the microorganism directly from the culture vessel. While the rate of supplying the culture medium was controlled such that the amount of culture liquid in the culture vessel was 1.5 L at a constant rate of collection of the culture liquid containing the microorganism, production of L-lactic acid was carried out for 300 hours (Table 13).

Culture vessel capacity: 2 (L)
Temperature adjustment: 50 (°C)
Kla: 30 (h$^{-1}$)
pH Adjustment: adjusted to pH 7 with 5 N Ca(OH)$_2$
Rate of collection of the fermentation liquid: 3 L/day
Sterilization: the fermentation vessel and media to be used were all subjected to high-pressure steam sterilization by autoclaving at 121°C for 20 min.

(Example 11) Production of L-Lactic Acid by Continuous Fermentation by *Bacillus coagulans* Using Mixed Sugar as Fermentation Feedstock, with Use of Separation Membrane 1

Using a mixed sugar as the fermentation feedstock, continuous fermentation was carried out with use of a separation membrane. The *Bacillus coagulans* NBRC12714 strain was cultured in 2 mL of a preculture medium (10 g/L polypeptone, 2 g/L yeast extract, 1 g/L magnesium sulfate 7H$_2$O, 30 g/L calcium carbonate) in a test tube at 50°C overnight (pre-pre-preculture). The obtained culture liquid was inoculated to 50 mL of a preculture medium placed in a 500-mL baffled Erlenmeyer flask, and culture was performed overnight (pre-preculture). The pre-preculture liquid was inoculated to the L-lactic acid fermentation medium (mixed sugar) having the composition shown in Table 9 placed in a continuous culture apparatus (the apparatus shown in Fig. 2 of WO2007/097260), and batch fermentation was carried out for 126 hours under the operating conditions shown below while the temperature and the pH were controlled (preculture). Immediately after completion of the preculture, continuous culture was started to produce L-lactic acid. For supplying the L-lactic acid fermentation medium (mixed sugar) 1 having the composition shown in Table 9 and filtering the culture liquid, a Perista BioMini Pump Type AC-2120 (ATTO) was used. The culture medium was directly supplied to the culture vessel, and the culture liquid was filtered through an element having an immobilized separation membrane. While the rate of supplying the culture medium was controlled such that the amount of culture liquid in the culture vessel was 1.5 L at a constant rate of filtration of the culture liquid, and while the transmembrane pressure difference during filtration was allowed to change within the range of 0.1 to 19.7 kPa, continuous fermentation was performed for 290 hours to produce L-lactic acid (Table 13).

Fermentation reaction vessel capacity: 2 (L)
Separation membrane used: polyvinylidene fluoride filtration membrane
Effective filtration area of the membrane separation element: 120 cm$^2$
Pure water permeation coefficient of the separation membrane: 50×10$^{-9}$ m$^3$/m$^2$/s/Pa

Average pore size of the separation membrane: 0.1±0.035 μm
Surface roughness of the separation membrane: 0.06 μm
Temperature adjustment: 50 (°C)
Kla: 30 (h⁻¹)
pH Adjustment: adjusted to pH 7 with 5 N Ca(OH)₂
Rate of collection of the fermentation liquid: 3 L/day
Sterilization: the fermentation vessel comprising the separation membrane element and media to be used were all subjected to high-pressure steam sterilization by autoclaving at 121°C for 20 min.

(Example 12) Production of L-Lactic Acid by Continuous Fermentation by *Bacillus coagulans* Using Mixed Sugar as Fermentation Feedstock, with Use of Separation Membrane 2

Using the *Bacillus coagulans* NBRC12714 strain, continuous fermentation was carried out with use of a separation membrane. The continuous fermentation was performed for 280 hours under the same conditions as in Example 11 except that Kla was set to 0, to produce L-lactic acid (Table 13).

(Example 13) Production of L-Lactic Acid by Continuous Fermentation by *Bacillus coagulans* Using Mixed Sugar as Fermentation Feedstock, with Use of Separation Membrane 3

Using the *Bacillus coagulans* NBRC12714 strain, continuous fermentation was carried out with use of a separation membrane. The continuous fermentation was performed for 270 hours under the same conditions as in Example 11 except that Kla was set to 60, to produce L-lactic acid (Table 13).

(Example 14) Production of L-Lactic Acid by Continuous Fermentation by *Bacillus coagulans* Using Mixed Sugar as Fermentation Feedstock, with Use of Separation Membrane 4

Using the *Bacillus coagulans* NBRC12714 strain, continuous fermentation was carried out with use of a separation membrane. The continuous fermentation was performed for 300 hours under the same conditions as in Example 1 except that Kla was set to 100, to produce L-lactic acid (Table 13).

(Example 15) Production of L-Lactic Acid by Continuous Fermentation by *Bacillus coagulans* Using Mixed Sugar as Fermentation Feedstock, with Use of Separation Membrane 5

Using the *Bacillus coagulans* NBRC12714 strain, continuous fermentation was carried out with use of a separation membrane. The continuous fermentation was performed for 290 hours under the same conditions as in Example 11 except that Kla was set to 150, to produce L-lactic acid (Table 13).

(Example 16) Continuous Culture of *Bacillus coagulans* Using Mixed Sugar as Fermentation Feedstock, with Use of Separation Membrane 6

Using the lactic acid fermentation medium (mixed sugar) 2 shown in Table 11, continuous culture was carried out for 305 hours under the same conditions as in Example 12 with use of a separation membrane, to produce L-lactic acid (Table 13).

[Table 11]
L-Lactic acid fermentation medium (mixed sugar) 2

| | |
|---|---|
| Glucose | 80 g |
| Xylose | 20 g |
| Yeast extract | 5 g |
| Ammonium sulfate | 1 g |
| K₂HPO₄ | 0.4 g |

Unit (1/Liter)

(Example 17) Continuous Culture of *Bacillus coagulans* Using Mixed Sugar as Fermentation Feedstock, with Use of Separation Membrane 6

Using the lactic acid fermentation mixed-sugar medium 3 shown in Table 12, continuous culture was carried out for 300 hours under the same conditions as in Example 12 with use of a separation membrane, to produce L-lactic acid (Table 13).

[Table 12]

L-Lactic acid fermentation medium (mixed sugar) 3

| | |
|---|---|
| Glucose | 20 g |
| Xylose | 80 g |
| Yeast extract | 5 g |
| Ammonium sulfate | 1 g |
| $K_2HPO_4$ | 0.4 g |

Unit (1/Liter)

[Table 13]

| | Comparative Example 7 | Comparative Example 8 | Comparative Example 9 | Example 11 | Example 12 | Example 13 | Example 14 | Example 15 | Example 16 | Example 17 |
|---|---|---|---|---|---|---|---|---|---|---|
| Fermentation period (hr) | 96 | 128 | 300 | 290 | 280 | 270 | 300 | 290 | 305 | 300 |
| Total glucose fed (g) | 100 | 50 | 1875 | 1813 | 1750 | 1688 | 1875 | 1813 | 3050 | 750 |
| Total xylose fed (g) | 0 | 50 | 1875 | 1813 | 1750 | 1688 | 1875 | 1813 | 763 | 3000 |
| Total production of L-lactic acid (g) | 60 | 53 | 1366 | 2621 | 2930 | 2286 | 2325 | 2103 | 3180 | 3150 |
| Unused glucose (g) | 0 | 0 | 0 | 0 | 0 | 0 | 0 | 0 | 0 | 0 |
| Unused xylose (g) | 0 | 0 | 1310 | 35 | 53 | 13 | 0 | 0 | 40 | 40 |
| Yield (g/g) | 0.6 | 0.53 | 0.56 | 0.73 | 0.85 | 0.68 | 0.62 | 0.58 | 0.84 | 0.85 |

(Comparative Example 10) Production of Ethanol by Batch Fermentation by *Escherichia coli* Using Biomass-derived Sugar Liquid as Fermentation Feedstock

Using a biomass-derived sugar liquid as the fermentation feedstock, fermentation was carried out. For preparation of the fermentation medium, a cellulose saccharification liquid prepared using a nanofiltration membrane by the preparation method disclosed in Example 2 of WO2010/067785 was used, and the composition of the fermentation medium was adjusted as shown in Table 14 using reagents as appropriate. Batch fermentation was performed for 24 hours under the same conditions as in Comparative Example 2 except that 4 N KOH was used for adjusting the pH, to produce ethanol (Table 17).

[Table 14]
Ethanol fermentation medium (sugar liquid) 1

| | |
|---|---|
| Glucose | 30 g |
| Xylose | 15 g |
| Yeast extract | 10 g |
| Tryptone | 5 g |
| NaCl | 5 g |

Unit (1/Liter)

(Comparative Example 11) Production of Ethanol by Continuous Fermentation by *Escherichia coli* Using Biomass-derived Sugar Liquid as Fermentation Feedstock

Using a biomass-derived sugar liquid as the fermentation feedstock, continuous fermentation was carried out without use of a separation membrane. For preparation of the fermentation medium, a cellulose saccharification liquid prepared using a nanofiltration membrane by the preparation method disclosed in Example 2 of WO2010/067785 was used. Reagents were used as appropriate to prepare the ethanol fermentation medium (sugar liquid) 1 shown in Table 14 as the preculture medium and the ethanol fermentation medium (sugar liquid) 2 shown in Table 15 as the culture medium to be used during continuous culture. Continuous fermentation was performed for 280 hours under the same conditions as in Comparative Example 3 except that 4 N KOH was used for adjusting the pH, to produce ethanol (Table 17).

[Table 15]
Ethanol fermentation medium (sugar liquid) 2

| | |
|---|---|
| Glucose | 60 g |
| Xylose | 30 g |
| Yeast extract | 10 g |
| Tryptone | 5 g |
| NaCl | 5 g |

Unit (1/Liter)

(Example 18) Production of Ethanol by Continuous Fermentation by *Escherichia coli* Using Biomass-Derived Sugar Liquid as Fermentation Feedstock, with Use of Separation Membrane

Using a biomass-derived sugar liquid as the fermentation feedstock, continuous fermentation was carried out with use of a separation membrane. Similarly to Comparative Example 17, the ethanol fermentation medium (sugar liquid) 1 shown in Table 14 was used as the preculture medium, and, similarly to Comparative Example 17, the ethanol fermentation medium (sugar liquid) 2 shown in Table 15 was used as the culture medium to be used during continuous culture. Continuous fermentation was performed for 290 hours under the same conditions as in Example 1 except that 4 N KOH was used for adjusting the pH, to produce ethanol (Table 17).

(Comparative Example 12) Production of L-Lactic Acid by Batch Fermentation by *Bacillus coagulans* Using Biomass-Derived Sugar Liquid as Fermentation Feedstock

Using a biomass-derived sugar liquid as the fermentation feedstock, fermentation was carried out. For preparation of the fermentation medium, a cellulose saccharification liquid prepared using a nanofiltration membrane by the preparation method disclosed in Example 2 of WO2010/067785 was used, and the composition of the fermentation medium was adjusted as shown in Table 16 using reagents as appropriate. Batch fermentation was performed for 70 hours under the same conditions as in Comparative Example 8 except that 4 N KOH was used for adjusting the pH and that Kla was set to 0 (h$^{-1}$), to produce L-lactic acid (Table 17).

[Table 16]

Lactic acid fermentation medium (sugar liquid)

| Glucose | 60 g |
|---|---|
| Xylose | 20 g |
| Yeast extract | 5 g |
| Ammonium sulfate | 1 g |
| $K_2HPO_4$ | 0.4 g |

Unit (1/Liter)

(Comparative Example 13) Production of L-Lactic Acid by Continuous Fermentation by *Bacillus coagulans* Using Biomass-Derived Sugar Liquid as Fermentation Feedstock

Using a biomass-derived sugar liquid as the fermentation feedstock, continuous fermentation was carried out without use of a separation membrane. Similarly to Comparative Example 12, the culture medium shown in Table 16 was used as the fermentation medium. Continuous fermentation was performed for 250 hours under the same conditions as in Comparative Example 9 except that 4 N KOH was used for adjusting the pH and that Kla was set to 0 (h$^{-1}$), to produce L-lactic acid (Table 17).

(Example 19) Production of L-Lactic Acid by Continuous Fermentation by *Bacillus coagulans* Using Biomass-Derived Sugar Liquid as Fermentation Feedstock, with Use of Separation Membrane

Using a biomass-derived sugar liquid as the fermentation feedstock, continuous fermentation was carried out with use of a separation membrane. Similarly to Comparative Example 12, the culture medium shown in Table 16 was used as the fermentation medium. Continuous fermentation was performed for 260 hours under the same conditions as in Example 11 except that 4 N KOH was used for adjusting the pH and that Kla was set to 0 (h$^{-1}$), to produce L-lactic acid (Table 17).

(Example 20) Production of 2,3-Butanediol by Continuous Fermentation by *Paenibacillus polymyxa* Using Mixed Sugar as Fermentation Feedstock, with Use of Ceramic Separation Membrane 6

Using the *Paenibacillus polymyxa* ATCC12321 strain, continuous fermentation was carried out using a ceramic separation membrane. The *Paenibacillus polymyxa* ATCC12321 strain was cultured in 2 mL of a preculture medium (5 g/L glucose, 5 g/L peptone, 3 g/L yeast extract, 3 g/L malt extract) in a test tube at 30°C overnight (pre-pre-preculture). The obtained culture liquid was inoculated to 50 mL of a preculture medium placed in a 500-mL baffled Erlenmeyer flask, and culture was performed overnight (pre-preculture). The pre-preculture liquid was inoculated to the 2,3-butanediol fermentation medium 1 (mixed sugar) having the composition shown in Table 5 placed in a continuous culture apparatus (the apparatus shown in Fig. 2 of WO2007/097260), and batch fermentation was carried out for 30 hours under the operating conditions shown below while the temperature and the pH were controlled (preculture). Immediately after completion of the preculture, continuous culture using the 2,3-butanediol fermentation medium 2 (mixed sugar) having the composition shown in Table 7 was started to produce 2,3-butanediol. While the transmembrane pressure difference during filtration was controlled at not more than 500 kPa, continuous fermentation was carried out for 260 hours to produce 2,3-butanediol (Table 17).

Fermenter capacity: 2 (L)
Separation membrane used: Celfit microfiltration membrane Monolith φ4-19 (NGK Insulators, Ltd.)
Length of the membrane separation element: 500 mm
Average pore size of the separation membrane: 0.1 μm
Temperature adjustment: 30 (°C)
Kla: 30 (h$^{-1}$)
pH Adjustment: adjusted to pH 6.5 with 5 N $Ca(OH)_2$
Rate of collection of the fermentation liquid: 0.7 L/day

[Table 17]

| | Comparative Example 10 | Comparative Example 11 | Example 18 | Comparative Example 12 | Comparative Example 13 | Example 19 | Example 20 |
|---|---|---|---|---|---|---|---|
| Fermentation period (hr) | 24 | 280 | 290 | 70 | 250 | 260 | 260 |
| Total glucose fed (g) | 30 | 1400 | 1450 | 60 | 1875 | 1950 | 303 |
| Total xylose fed (g) | 15 | 700 | 725 | 20 | 625 | 650 | 607 |
| Total production of ethanol (g) | 12 | 304 | 835 | | | | |
| Total production of L-lactic acid (g) | | | | 52 | 1254 | 2176 | |
| Total production of 2,3-butanediol (g) | | | | | | | 273 |
| Unused glucose (g) | 0 | 0 | 0 | 0 | 0 | 0 | 0 |
| Unused xylose (g) | 0 | 380 | 87 | 0 | 410 | 40 | 0 |
| Yield (g/g) | 0.27 | 0.23 | 0.4 | 0.65 | 0.60 | 0.85 | 0.30 |

EP 2 803 731 A1

As shown by the above results, chemical products could be produced with high yield from a mixed sugar of pentose and hexose.

INDUSTRIAL APPLICABILITY

By the present invention, the efficiencies of fermentation production of various chemical products using a fermentation feedstock containing pentose and hexose can be largely increased.

## Claims

1. A method for producing a chemical product by continuous fermentation, said method comprising filtering a culture liquid of a microorganism(s) through a separation membrane; retaining unfiltered liquid in, or refluxing unfiltered liquid to, the culture liquid; adding a fermentation feedstock to the culture liquid; and recovering a product in the filtrate, wherein said fermentation feedstock comprises pentose and hexose, and wherein said microorganism(s) has/have a pathway in which pentose isomerase is used to metabolize pentose.

2. The method for producing a chemical product according to claim 1, comprising performing continuous fermentation under conditions where the oxygen transfer coefficient (Kla) is not more than 150 h$^{-1}$.

3. The method for producing a chemical product according to claim 1 or 2, wherein the weight ratio between the hexose and the pentose contained in said fermentation feedstock is 1:9 to 9:1.

4. The method for producing a chemical product according to claim 1 or 2, wherein said fermentation feedstock comprises a biomass-derived sugar liquid.

5. The method for producing a chemical product according to any one of claims 1 to 4, wherein said pentose isomerase is xylose isomerase.

6. The method for producing a chemical product according to any one of claims 1 to 5, wherein said pentose is xylose.

# EP 2 803 731 A1

## INTERNATIONAL SEARCH REPORT

| | International application No. |
|---|---|
| | PCT/JP2013/050438 |

**A. CLASSIFICATION OF SUBJECT MATTER**
*C12P7/06*(2006.01)i, *C12P7/18*(2006.01)i, *C12P7/56*(2006.01)i

According to International Patent Classification (IPC) or to both national classification and IPC

**B. FIELDS SEARCHED**

Minimum documentation searched (classification system followed by classification symbols)
C12P7/06, C12P7/18, C12P7/56

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY/MEDLINE/EMBASE/BIOSIS/WPIDS(STN),
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| X | JP 4770987 B2 (Toray Industries, Inc.), 14 September 2011 (14.09.2011), paragraphs [0122], [0208]; examples 1, 17 (Family: none) | 1-6 |
| Y | WO 2007/097260 A1 (Toray Industries, Inc.), 30 August 2007 (30.08.2007), claims; paragraphs [0003] to [0012] & JP 2007-252367 A & US 2009/0269812 A1 & EP 1988170 A1 & CA 2638780 A & KR 10-2008-0096710 A | 1-6 |
| Y | KRISHNAN et al., Ethanol production from glucose and xylose by immobilized Zymomonas mobilis CP4 (pZB5), Applied Biochemistry and Biotechnology, 2000, Vol.84-86, pp.525-541 | 1-6 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 08 February, 2013 (08.02.13) | 19 February, 2013 (19.02.13) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2013/050438 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | TANAKA et al., Two different pathways for D-xylose metabolism and the effectof xylose concentration on the yield coefficient of L-lactate in mixed-acidfermentation by the lactic acid bacterium Lactococcus lactis IO-1, Appl Microbiol Biotechnol, 2002, Vol.60, pp.160-167 | 1-6 |
| A | ERLANDSON et al., Dissolution of Xylose Metabolism inLactococcus lactis, Appl. Environ. Microbiol., 2000, Vol.66, No.9, pp.3974-3980 | 1-6 |
| A | JP 2009-28030 A  (Gekkeikan Sake, Co., Ltd.), 12 February 2009 (12.02.2009), paragraph [0013] (Family: none) | 2-6 |
| A | JP 2011-139707 A  (Nippon Suisan Kaisha, Ltd.), 21 July 2011 (21.07.2011), paragraph [0025] & US 2005/0287651 A1      & EP 1549753 A & WO 2004/033698 A2      & CA 2501880 A & KR 10-2005-0055761 A | 2-6 |
| A | FOND et al., The acetone butanol fermentation on glucose and xylose. I. Regulation and kinetics in batch cultures, Biotechnology and Bioengineering, 1986, Vol.XXVIII, pp.160-166 | 1-6 |
| A | KIM et al., Batch and continuous fermentation of succinic acid from wood hydrolysate by Mannheimia succiniciproducens MBEL55E, Enzyme and Microbial Technology, 2004, Vol.35, pp.648-653 | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010067785 A
- JP H11506934 A
- JP 2005229821 A
- JP 2003212888 A
- JP 2001095597 A
- JP 3041380 B
- JP 2008079564 A
- JP 2006525029 A
- WO 2007097260 A
- WO 2012086763 A

**Non-patent literature cited in the description**

- **KAISA KARHUMAA ; ROSA GARCIA SANCHEZ ; BARBEL HAHN-HAGERDAL ; MARIE-F GORWA-GRAUSLUND.** Comparison of the xylose reductase-xylitol dehydrogenase and the xylose isomerase pathways for xylose fermentation by recombinant Saccharomyces cerevisiae. *Microbial Cell Factories,* 2007, vol. 6, 5
- **DO YUN KIM ; SEONG CHUN YIM ; PYUNG CHEON LEE ; WOO GI LEE ; SANG YUP LEE ; HO NAM CHANG.** Batch and continuous fermentation of succinic acid from wood hydrolysate by Mannheimia succiniciproducens MBEL55E. *Enzyme and Microbial Technology,* 2004, vol. 35, 648-653
- **A. ADEN et al.** Lignocellulosic Biomass to Ethanol Process Design and Economics Utilizing Co-Current Dilute Acid Prehydrolysis and Enzymatic Hydrolysis for Corn Stover. *NREL Technical Report,* 2002
- Laboratory Manual for Bioengineering. Baifukan Co., Ltd, 1992, 310